# EUROPÄISCHE PATENTSCHRIFT

(11) **EP 0 550 448 B1**
(45) Veröffentlichungstag und Bekanntmachung des Hinweises auf die Patenterteilung: **25.05.1994**
(21) Anmeldenummer: 91914055.8
(22) Anmeldetag: 30.07.1991
(51) Int. Cl.: C07H 3/02, C07H 3/08

(54) **VERFAHREN ZUR HERSTELLUNG VON RHAMNOSE AUS RHAMNOLIPIDEN**
PROCESS FOR PRODUCING RHAMNOSE FROM RHAMNOLIPIDS
PROCEDE DE FABRICATION DE RHAMNOSE A PARTIR DE RHAMNOLIPIDES

(30) Priorität: 25.09.1990 DE 4030262
(43) Veröffentlichungstag der Anmeldung: 14.07.1993
(73) Patentinhaber: SÜDZUCKER AKTIENGESELLSCHAFT MANNHEIM/OCHSENFURT, D-68165 Mannheim (DE)
(72) Erfinder: MIXICH, Johann, D-6233 Kelkheim (DE); RAPP, Knut, Martin, D-6521 Offstein (DE); VOGEL, Manfred, D-6719 Neuleiningen (DE)
(74) Vertreter: Gleiss & Grosse
(86) Internationale Anmeldenummer: EP9101426
(87) Internationale Veröffentlichungsnummer: WO9205182

(56) Entgegenhaltungen:
- EP-A- 0 314 959
- EP-A- 0 317 036
- Biotechnology and Bioengineering, Vol.33,No.3, 15 January 1989, J. Wiley & Sons , (New York, US), R.J. Linhardt et al.: " Microbially produced Rhamnolipid as a source of Rhamnose" , pages 365-368, see page 366,column 2

## Beschreibung

Rhamnose ist ein 6-Desoxyzucker (Monosaccharid), der in der Natur sowohl in der D- als auch in der L-Form vorkommt.

Die Herstellung von L-Rhamnose, die das heute am einfachsten zugängliche 6-Desoxymonosaccharid ist, ist Gegenstand verschiedener Patentanmeldungen. Aus derartigen Desoxyzuckern läßt sich u.a. 2,5-Dimethyl-4-hydroxy-2,3-dihydrofuran-3-on (Furaneol) herstellen, das Verwendung als Aromastoff findet.

Als Ausgangsstoffe für eine Gewinnung von Rhamnose kommen verschiedene Naturstoffe in Frage, z.B. Glycoside wie Rutin, Quercitrin, Naringin, Hesperidin und Polysaccharide wie Gummi arabicum, das fermentativ gewonnene Polysaccharid S-60 (US-PS 4 326 053) oder Rhamnolipide, die fermentativ oder mikrobiell, z.B. von Bakterien der Gattung Pseudomonas, u.a. aus natürlichen Ölen oder Erdölfraktionen produziert werden.

Unabhängig von der Art der vorgenannten Ausgangsstoffe ist zur Gewinnung der Rhamnose eine Hydrolyse, die enzymatisch oder sauer katalysiert sein kann, notwendig, wobei man ein Gemisch enthält, das neben Rhamnose noch andere Substanzen enthält.

Wenn man von Rhamnose enthaltenden Glycosiden ausgeht, ist man auf pflanzliche Ausgangsprodukte, z.B. Abfälle der Zitrussaftherstellung angewiesen, die nur im jahreszeitlichen Rhythmus anfallen und deren Zusammensetzung in weiten Grenzen schwankt. Um von derartigen Schwankungen unabhängig zu sein, ist es vorteilhafter, fermentativ gewonnene Polysaccharide oder Rhamnolipide als Ausgangsstoffe zu verwenden, die reproduzierbar hergestellt werden können.

Die Verwendung von Rhamnolipiden hat gegenüber der von Rhamnose enthaltenden Heteropolysacchariden den Vorteil, daß der gewünschte Zucker - Rhamnose - nach einer Hydrolyse nicht von anderen Zuckern, z.B. Glucose und Mannose, die sonst vielfach in wesentlichen Mengen in der Hydrolyselösung enthalten sind, abgetrennt werden muß. Die Trennung eines komplexen Zuckergemisches ist technisch aufwendiger als die Isolierung der Rhamnose aus dem Hydrolysat eines Rhamnolipids.

Ein Rhamnolipid, insbesondere ein fermentativ gewonnenes Rhamnolipid, ist somit ein gut geeignetes Ausgangsmaterial für eine Rhamnose-Produktion.

Für eine Produktion von Rhamnose in technischem Maßstab ist neben einem geeigneten Ausgangsmaterial die Möglichkeit einer leichten Isolierung der Rhamnose aus einem Hydrolysat komplexer Zusammensetzung von ganz entscheidender Bedeutung.

Für die Gewinnung von Rhamnose aus Hydrolysaten sind folgende Methoden beschrieben:

Nach der DE-OS 35 45 107 wird zu dem neutralisierten wäßrigen Hydrolysat eine große, insbesondere eine 5- bis 20-fache Menge, bezogen auf die wäßrige Phase, eines polaren organischen Lösungsmittels zugegeben. Danach erfolgt die Entfernung des Lösungsmittels, Auftrennung der Zucker über stark saure Kationenaustauscher, vorzugsweise unter Verwendung von Aceton oder Acetonitril als "Extraktionsmittel", und Reinigung der Rhamnose durch Adsorption an Aktivkohle. Dieses Aufarbeitungsverfahren ist umständlich und für eine wirtschaftliche Gewinnung von Rhamnose nicht geeignet.

Nach der EP-A 317 033 werden in Zitrusabfällen enthaltene Glycoside enzymatisch zur Gewinnung von Rhamnose hydrolysiert. Die im Hydrolysat enthaltene Glucose wird durch Vergären mit Hefe oder durch selektive Oxidation der Glucose zur 5-Ketogluconsäure entfernt. Diese Methode ist teuer und umständlich, zumal noch eine chromatographische Reinigung über Aktivkohle erforderlich ist.

Nach der EP-A 282 942 wird ein isoliertes Rhamnolipid als Ausgangsmaterial verwendet, das mit H₂SO₄ bei 30 bis 100°C hydrolysiert wird. Die dabei entstehende Hydroxydecansäure wird entweder mit Ethylacetat extrahiert oder an Anionenaustauschern adsorbiert. Die Hydrolyse des Rhamnolipid-Niederschlages (Bsp. III) erfolgt in sehr verdünnter Lösung. 1,9 g Rhamnose enthaltender Niederschlag, entsprechend ca. 3,8 g Rhamnolipid, werden in 300 ml einmolarer H₂SO₄ suspendiert, erhitzt und anschließend mit dem vierfachen Volumen, d.i. 1200 ml Ethylacetat, behandelt. In der wäßrigen Phase werden 1,2 g Rhamnose gefunden, in der organischen (Ethylacetat-)-Phase verbleiben 0,5 g Rhamnose.

Die für die Herstellung von nur 1,2 g Rhamnose erforderlichen Säure- und Lösungsmittelmengen bedingen, daß eine wirtschaftliche Produktion von Rhamnose auf diese Weise nicht möglich ist.

Es ist also bisher keine Methode bekannt, die es erlaubt, Rhamnose in großer Menge mit wirtschaftlichen Mitteln und ohne teure und gefährliche Hilfsstoffe (Enzyme, brennbare und giftige Lösungsmittel) herzustellen.

Gegenstand der Erfindung ist nun ein Verfahren zur Herstellung von Rhamnose aus Rhamnolipiden, das dadurch gekennzeichnet ist, daß man eine saure Emulsion des Rhamnolipids bei 100 bis 200°C hydrolysiert und anschließend kühlt, die wäßrige Phase des entstandenen Hydrolysats von der lipiden Phase abtrennt, ihren pH-Wert durch Zugabe einer basischen Verbindung anhebt, einen etwa entstehenden Niederschlag abtrennt, die verbleibende Lösung einengt und entweder unmittelbar weiterverarbeitet oder über ein Ionenaustauscherharz chromatographiert, wobei als Eluat Rhamnose enthaltende Fraktionen anfallen, die als solche weiterverarbeitet oder zu kristallinem Rhamnosemonohydrat aufgearbeitet werden.

Es ist ein Vorteil des erfindungsgemäßen Verfahrens, daß man auf die Mitverwendung von organischen Lösungsmitteln vollständig verzichten und als Lösungsmittel ausschließlich Wasser verwenden kann.

Die bei dem erfindungsgemäßen Verfahren verwendeten Rhamnolipide lassen sich auf verschiedene Weise, z.B. gemäß der EP-A 282 942 und der DE-A 34 05 664 nach fermentativen Verfahren herstellen. Zweckmäßig werden die erhaltenen Emulsionen weitgehend von Fremdsalzen befreit, vorteilhaft soweit, daß sie eine Leitfähigkeit von weniger als 12 mS/cm, vorzugsweise von weniger als 5 mS/cm aufweisen. Derartige entsalzte Emulsionen können auch nach dem Verfahren der am gleichen Tage eingereichten Patentanmeldung der Hoechst Aktiengesellschaft (HOE 90/F 284) mit dem Titel "Verfahren zur Herstellung geeigneter Glycolipide durch Membrantrennverfahren durch Ultrafiltration hergestellt werden. Für das erfindungsgemäße Verfahren werden zweckmäßig solche Emulsionen verwendet, deren wäßrige Phase einen pH-Wert von 0 bis 3, vorzugsweise von 0,5 bis 1,5 aufweisen und die einen Trockensubstanzgehalt, d.i. Verdampfungsrückstand durch 30 minütiges Erhitzen auf 85°C bei 6 kPa bestimmt, von 5 bis 50, vorzugsweise 10 bis 20 Gew.-% aufweisen. Dieser Trockensubstanzgehalt läßt sich durch Aufkonzentrierung bzw. Verdünnung mit Wasser einstellen.

Das erfindungsgemäße Verfahren kann diskontinuierlich oder kontinuierlich durchgeführt werden, wobei die kontinuierliche Arbeitsweise bevorzugt ist.

Für die Einstellung der Emulsion auf den pH-Wert von 0 bis 3 kann man z.B. Salzsäure verwenden; ist jedoch kontinuierliches Arbeiten beabsichtigt, empfiehlt es sich, als Säuren solche zu verwenden, die mit der basischen Verbindung, die im späteren Verlaufe des Verfahrens zugesetzt wird, ein schwerlösliches Salz bilden, vorzugsweise H₂SO₄, H₃PO₄ und HF.

Die saure Emulsion wird z.B. diskontinuierlich oder kontinuierlich unter gutem Rühren 5 bis 300 min, vorzugsweise 20 bis 150 min, insbesondere 30 bis 120 min auf eine Temperatur von 100 bis 200°C, vorzugsweise 110 bis 160°C, insbesondere 120 bis 150°C erhitzt und anschließend gekühlt. Eine gute Durchmischung des zu hydrolysierenden Gemisches bis zur Trennung der wäßrigen Phase von der Ölphase ist sehr wichtig, da es sich um eine heterogene Hydrolyse handelt, bei der sich die als Katalysator wirkende Säure sowie die freigesetzte Rhamnose in der Wasserphase befinden, und das zu hydrolysierende Rhamnolipid zusammen mit nicht umgesetztem Substrat, z.B. Sojaöl in der Lipidphase. Die Durchmischung kann durch physikalische Mittel unterstützt werden, beispielsweise durch Rühren, Schütteln oder Anwendung von Ultraschall.

Die Dauer der Hydrolyse ist wie beim diskontinuierlichen Verfahren natürlich von der angewandten Temperatur abhängig. Bei der kontinuierlichen Arbeitsweise läßt sich die Aufenthaltszeit über die Änderung verschiedener Faktoren variieren, wie z.B. der Leistung der Einspeisepumpe, der Zahl der Reaktoren bzw. dem Volumen bis zum Druckhalteventil.

Nach der Hydrolyse fällt ein Produkt an, das im wesentlichen aus einer wäßrigen Phase besteht, die die freigesetzte Rhamnose enthält und neben der noch eine Ölphase vorhanden ist, die u.a. unumgesetztes Rhamnolipid enthält. Dieses Produkt wird z.B. in einem Separator in die beiden Phasen getrennt. Um eine leichte Trennung zu ermöglichen, werden die beiden Phasen bei einer Temperatur von unter 100°C, vorzugsweise bei etwa 50 bis 90°C, separiert.

Als basische Verbindungen eignen sich insbesondere solche, die mit der eingesetzten Säure ein schwerlösliches Salz bilden, vorzugsweise Ca(OH)₂ und/oder CaCO₃. Derartige Salze sind vor allem für die kontinuierliche Arbeitsweise bevorzugt. Die Anhebung des pH-Wertes erfolgt zweckmäßig bis zu einem Wert von 2 bis 8, vorzugsweise 3 bis 7. Der gebildete Feststoff wird, gegebenenfalls unter Zusatz eines Filterhilfsmittels, abgetrennt und das Filtrat unter vermindertem Druck, vorzugsweise bei unter 20 kPa, z.B. unter 60°C, auf einen Trockensubstanzgehalt von 20 bis 60, vorzugsweise 25 bis 50 Gew.-% eingeengt. Als Filterhilfsmittel kommen z.B. solche auf Basis von Zellulose oder Kieselgur, beispielsweise ®Arbocell und ®Celite in Frage.

Geeignete Ionenaustauscherharze sind sulfonierte mit Divinylbenzol vernetzte Polystyrolharze in der Salzform, die vorzugsweise dieselbe Art von Kationen enthalten wie die zur Neutralisation verwendete basische Verbindung. Die bei der Chromatographie erhaltenen Rhamnose enthaltenden Fraktionen werden unter vermindertem Druck, vorzugsweise bei unter 20 kPa, weiter in Abhängigkeit von der Temperatur eingeengt, z.B. auf einen Trotkensubstanzgehalt von ca. 65 Gew.-%, und nach einer etwaigen Filtration kristallisiert.

Die Rhamnose kann vor oder nach einer chromatographischen Trennung in dieser Form bereits weiterverarbeitet werden, z.B. zu Furaneol, wenn die Reinheit der Lösung ausreichend ist.

Zur Vorreinigung der wäßrigen Phase vor der Chromatographie und der Kristallisation kann diese mit Entfärbungsmitteln, wie z.B. Aktivkohle oder Bentonit behandelt werden.

Die Kristallisation kann als Verdampfungs- und/oder Kühlungskristallisation durchgeführt werden. Die Kühlungskristallisation erfolgt bevorzugt zwischen 65 und 15°C mit einer Kühlrate von 1 bis 10°C/h, vorzugsweise 3 bis 6°C/h. Die Verdampfungskristallisation läßt sich beispielsweise so durchführen, daß man bei einer Temperatur von 65°C und einem Druck von 20 kPa Wasser so schnell verdampft, daß eine Übersättigung in der Mutterlauge von 1,05 bis 1,3, vorzugsweise 1,1 bis 1,15 eingehalten wird und die Kristalle von Rhamnosemonohydrat bis zu einer Kantenlänge von 0,3 bis 0,5 mm wachsen können. Das Kristallisat kann in einer Siebkorbzentrifuge von der Mutterlauge abgetrennt werden. Die Mutterlauge kann einer zweiten Kristallisationsstufe unterworfen werden. Eine solche mehrstufige Verdampfungskristallisation führt bekanntlich zu einer höheren Ausbeute an kristallisiertem Produkt.

Die danach anfallende Restmutterlauge kann durch eine chromatographische Behandlung von störenden Fremdsubstanzen befreit und noch weiter wie oben beschrieben zu kristallinem Rhamnosemonohydrat aufgearbeitet werden.

Als Rohprodukt fällt die Rhamnose in Form von Kristallen von Rhamnosemonohydrat (Reinheit ca. 95 %) an. Dieses Rohkristallisat kann aus Wasser umkristallisiert werden und in üblicher Weise, z.B. unter Bewegung mit über 70°C warmer Luft oder unter vermindertem Druck, beispielsweise unter 20 kPa, bei einer Temperatur von 20 bis 70°C, getrocknet werden.

Das Verfahren einer kontinuierlichen sauren Hydrolyse eines Rhamnolipids wird beispielhaft im folgenden genauer beschrieben.

### Beispiele

1. Aus einem fermentativ erhaltenen entsalzten Konzentrat mit einem Trockensubstanzgehalt von 40 Gew.-% (gravimetrisch bestimmt) wurde unter Rühren mit Schwefelsäure und Wasser eine Rhamnolipidemulsion mit 15 Gew.-% Trockensubstanzgehalt und einem pH-Wert von 0,9 hergestellt.
   380 l dieser Emulsion wurden stündlich mittels einer Mohnopumpe in eine Hydrolyseanlage eingebracht, die im wesentlichen besteht aus:
   a) einer Homogenisieranlage (z.B. ®SUPRATON-Maschine) mit einer geregelten Dampfinjektion in der Saugseite,
   b) einer Heißhaltestrecke, bestehend aus mehreren hintereinander zu durchlaufenden Reaktoren, deren letzter eine Druckhalteeinrichtung (Druckhalteventil) besitzt, womit ein bestimmter Druck zwischen der Mohnopumpe und dem Druckhalteventil eingestellt werden kann, in der die wäßrige Emulsion auf 150 bis 160°C erhitzt wird, wobei die Aufenthaltszeit in der Heißhaltestrecke etwa 120 min beträgt, und
   c) einer Kühleinrichtung, z.B. Röhrenkühler, mit der das Hydrolysat unter 100°C abgekühlt wird.

   In der SUPRATON-Maschine sind Werkzeuge eingebaut, die eine gute Homogenisierung und Zerkleinerung bewirken. Diese Maschine hat die Aufgabe, den Produktstrom hohen Scherkräften zu unterwerfen und somit Säure, Wasser und das Rhamnolipid innig zu durchmischen, Bedingungen, die für ein heterogenes Reaktionsgemisch von großer Bedeutung sind.
   Das gekühlte Hydrolysat wurde in einem mit einem Rührer versehenen Behälter gesammelt. Dann wurden die Phasen mechanisch, z.B. in einem Separator (z.B. Westfalia-Separator) getrennt und die wäßrige Phase kontinuierlich abgezogen. Diese wurde in einem mit einem Rührer versehenen Behälter gesammelt, mit Calciumcarbonat auf einen pH-Wert von ca. 4,5 gebracht und der entstandene Niederschlag (Gips), z.B. in einer Kammerfilterpresse, abgetrennt, gewaschen und das Filtrat unter vermindertem Druck (unter 20 kPa) eingeengt. Man erhält aus insgesamt 380 l/h der in die Hydrolyseanlage eingespeisten Emulsion etwa 440 l/h Hydrolysat und etwa 380 l/h Filtrat. Dieses wurde auf etwa 50 Gew.-% Trockensubstanzgehalt eingeengt, filtriert und über einen Ionenaustauscher Lewatit TSW 40 in Kalziumform chromatographiert.
   Die zur chromatographischen Trennung verwendete Anlage besteht aus 3 Säulen (1 m Durchmesser) mit einem Gesamt-Harz-Inhalt von 14 m³ Harz.
   700 kg eines Rohproduktes mit einem Trockensubstanzgehalt von 47,6 Gew.-% (333 kg Trockensubstanz), die 244 kg Rhamnose enthalten, werden bei 65°C und einem Durchfluß von 1,6 m³/h mit entsalztem Wasser eluiert. Die Produktfraktion, die nach 0,5 l Bettvolumen am Ende der 3. Säule enfällt und 0,275 l Bettvolumen beträgt (3,85 m³, 210 kg Rhamnose enthaltend), wird schonend eingeengt und gemeinsam mit einer weiteren Produktfraktion zur Kristallisation gebracht.
   660 kg der konzentrierten Produktfraktion (TS-Gehalt 69,2 Gew.-%, 457 kg TS) werden in einem 600 l Kühlungskristallisator unter Rühren von 65°C auf 20°C mit einer Kühlrate von 3°C/h abgekühlt und anschließend in einer Siebkornzentrifuge in 354 kg Rhamnosemonohydratkristalle und 386 kg Mutterlauge (incl. Waschwasser) getrennt.
   Die Kristalle haben eine Reinheit von 98 % und sind schwach gelb gefärbt. Sie können aus Wasser umkristallisiert werden. Die Mutterlauge wird einer weiteren Kristallisation unterworfen und die daraus resultierende Endmutterlauge wird rechromatographiert.
2. (Diskontinuierliche Hydrolyse) Zu einem durch Ultrafiltration entsalzten Konzentrat (40 % TS) wurde konzentrierte Salzsäure in einer Menge zugegeben, so daß die gesamte Mischung ca. 1/8 normal an Salzsäure war (0,46 Gew.-%).
   In einem Rühr-Autoklav wurde die Emulsion zwei Stunden auf 140°C erhitzt, nach dem Abkühlen erfolgte eine Phasenseparierung. Die wäßrige Phase wurde mit Calciumhydroxyd auf einen pH-Wert von etwa 6 bis 8 eingestellt, nach Zugabe von Bentonit eine Stunde gerührt und filtriert.
   Das Filtrat hat einen pH-Wert von 6 bis 8, einen Trockensubstanzgehalt von ca. 11 Gew.-% und enthält ca. 75 g Rhamnose/l. Diese Lösung wurde direkt zur Kristallisation gebracht. Die Mutterlauge wurde wie in Beispiel 1 chromatographiert und wie in Beispiel 1 oder 3 zur Kristallisation gebracht.
3. (Verdampfungskristallisation) In einem Verdampfungskristallisator (100 l Inhalt) wurde isobar bei 20 kPa die konzentrierte Produktfraktion mit einem TS-Gehalt von ca. 65 Gew.-% eingeengt, wobei das Niveau über der Heizkammer gehalten wurde. Bei einer Übersättigung von 1,1 (s. Abb. Löslichkeitskurve) wurde mit feingemahlenen Rhamnosemonohydratkristallen angeimpft und anschließend die konzentrierte Produktlösung entsprechend der Kristallisationsgeschwindigkeit langsam nachgezogen, bis der Kochapparat gefüllt war. Der TS-Gehalt des Magmas beträgt dann ca. 80 Gew.-%. Die Füllmasse (Magma) wurde anschließend in einer Siebkorbzentrifuge in 27,8 kg Kristalle von Rhamnosemonohydrat und 46,5 kg Mutterlauge getrennt.
   Die Mutterlauge wurde ein zweites Mal kristallisiert; die Endmutterlauge wurde in einer chromatographischen Trennung von Nebenprodukten befreit und die Rhamnose wie oben beschrieben kristallisiert.

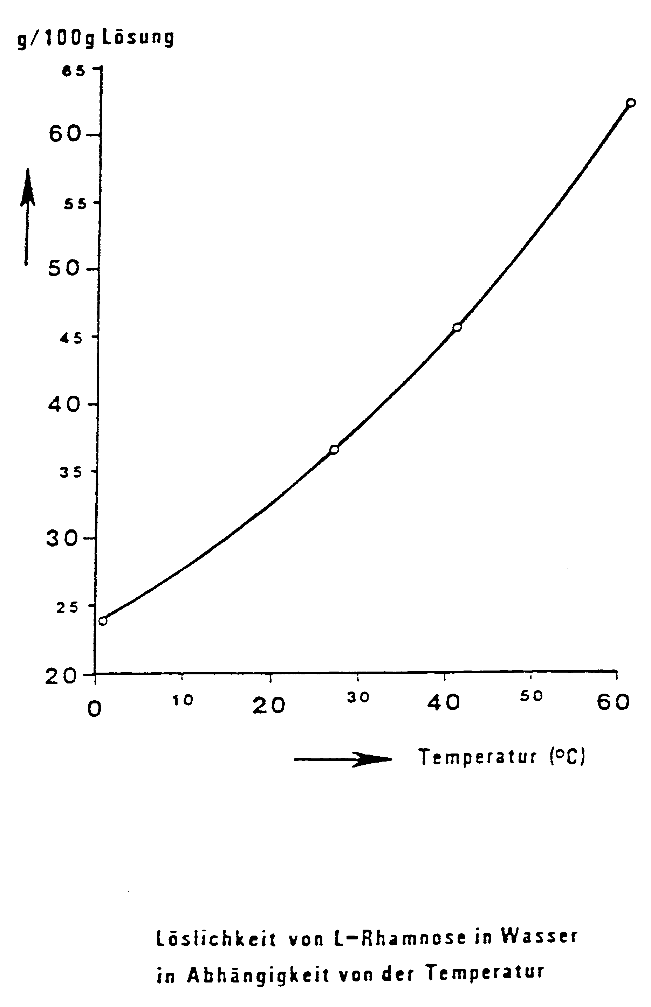

## Patentansprüche

1. Verfahren zur Herstellung von Rhamnose aus Rhamnolipiden, dadurch gekennzeichnet, daß man eine saure Emulsion des Rhamnolipids bei 100 bis 200°C hydrolysiert und anschließend kühlt, die wäßrige Phase des entstandenen Hydrolysats von der lipiden Phase abtrennt, ihren pH-Wert durch Zugabe einer basischen Verbindung anhebt, einen etwa entstehenden Niederschlag abtrennt, die verbleibende Lösung einengt, und entweder unmittelbar weiterverarbeitet oder über ein Ionenaustauscherharz chromatographiert, wobei als Eluat Rhamnose enthaltende Fraktionen anfallen, die als solche weiterverarbeitet oder zu kristallinem Rhamnosemonohydrat aufgearbeitet werden.

2. Verfahren nach Anspruch 1, dadurch gekennzeichnet, daß als Lösungsmittel ausschließlich Wasser verwendet wird.

3. Verfahren nach Anspruch 1 oder 2, dadurch gekennzeichnet, daß man von einer fermentativ gewonnenen Emulsion von Rhamnolipiden ausgeht.

4. Verfahren nach einem oder mehreren der Ansprüche 1 bis 3, dadurch gekennzeichnet, daß man kontinuierlich arbeitet.

5. Verfahren nach einem oder mehreren der Ansprüche 1 bis 4, dadurch gekennzeichnet, daß die lipide Phase spätestens in der Hydrolysestufe in das Verfahren zurückgeführt wird.

6. Verfahren nach einem oder mehreren der Ansprüche 1 bis 5, dadurch gekennzeichnet, daß man eine Rhamnolipid-Emulsion verwendet, die eine Leitfähigkeit von weniger als 12 mS/cm vorzugsweise von weniger als 5 mS/cm aufweist.

7. Verfahren nach einem oder mehreren der Ansprüche 1 bis 6, dadurch gekennzeichnet, daß die Emulsion einen Trockensubstanzgehalt von 5 bis 50 Gew.-%, vorzugsweise 10 bis 20 Gew.-%, aufweist.

8. Verfahren nach einem oder mehreren der Ansprüche 1 bis 7, dadurch gekennzeichnet, daß die wäßrige Phase der sauren Emulsion einen pH-Wert von 0 bis 3, vorzugsweise 0,5 bis 1,5 aufweist.

9. Verfahren nach einem oder mehreren der Ansprüche 1 bis 8, dadurch gekennzeichnet, daß die Hydrolyse bei 110 bis 160°C, vorzugsweise bei 120 bis 150°C durchgeführt wird.

10. Verfahren nach einem oder mehreren der Ansprüche 1 bis 9, dadurch gekennzeichnet, daß die wäßrige Phase des entstandenen Hydrolysats bei unter 100°C, vorzugsweise bei 50 bis 90°C, von der lipiden Phase abgetrennt wird.

11. Verfahren nach einem oder mehreren der Ansprüche 1 bis 10, dadurch gekennzeichnet, daß man den pH-Wert der wäßrigen Phase durch Zugabe einer basischen Verbindung, die mit einem in der Lösung enthaltenen Säureanion ein schwerlösliches Salz bildet, vorzugsweise Ca(OH)₂ und/oder CaCO₃, auf 2 bis 8, vorzugsweise 3 bis 7, anhebt.

12. Verfahren nach einem oder mehreren der Ansprüche 1 bis 11, dadurch gekennzeichnet, daß man die nach der Abtrennung des Niederschlags verbleibende Lösung mit einem Entfärbungsmitter, vorzugsweise Bentonit, behandelt.

13. Verfahren nacheinem oder mehreren der Ansprüche 1 bis 12, dadurch gekennzeichnet, daß die Lösung, die über das Ionenaustauscherharz chromatographiert wird, auf einen Trockensubstanzgehalt von 20 bis 60, vorzugsweise 25 bis 50 Gew.-% eingeengt wird.

14. Verfahren nach einem oder mehreren der Ansprüche 1 bis 13, dadurch gekennzeichnet, daß die Kristallisation des Rhamnosemonohydrats als Kühlungs- oder Verdampfungskristallisation durchgeführt wird.

15. Verfahren nach Anspruch 14, dadurch gekennzeichnet, daß die Kühlungskristallisation mit einer Kühlrate von 1 bis 10, vorzugsweise 3 bis 6°C/h durchgeführt wird.

16. Verfahren nach Anspruch 14, dadurch gekennzeichnet, daß bei der Verdampfungskristallisation eine Übersättigung in der Mutterlauge von 1,05 bis 1,3, vorzugsweise 1,1 bis 1,15, eingehalten wird.

## Claims

1. Method for the preparation of rhamnose from rhamnolipids, characterised in that an acid emulsion of rhamnolipid is hydrolysed at 100 to 200°C and subsequently cooled, the aqueous phase of the resulting hydrolysate is separated from the lipid phase, its pH value is raised by the addition of a basic compound, any resulting precipitate is separated, the remaining solution is concentrated and either further processed directly or chromatographed by way of an ion exchanger resin, wherein fractions containing rhamnose accumulate as the eluate, which fractions are further processed as such or are processed to form crystalline rhamnose monohydrate.

2. Method according to claim 1, characterised in that water is used exclusively as the solvent.

3. Method according to claim 1 or 2, characterised in that the procedure is carried out from an emulsion of rhamnolipids obtained by fermentation.

4. Method according to one or more of claims 1 to 3, characterised in that work is continuous.

5. Method according to one or more of claims 1 to 4, characterised in that the lipid phase is led back to the method at the hydrolysis step at the latest.

6. Method according to one or more of claims 1 to 5, characterised in that-a rhamnolipid emulsion which has a conductivity of less than 12mS/cm, preferably less than 5mS/cm, is used.

7. Method according to one or more of claims 1 to 6, characterised in that the emulsion has a dry substance content of 5 to 50% by weight, preferably 10 to 20% by weight.

8. Method according to one or more of claims 1 to 7, characterised in that the aqueous phase of the acid emulsion has a pH value of 0 to 3, preferably 0.5 to 1.5.

9. Method according to one or more of claims 1 to 8, characterised in that hydrolysis is carried out at 110 to 160°C, preferably at 120 to 150°C.

10. Method according to one or more of claims 1 to 9, characterised in that the aqueous phase of the resulting hydrolysate is separated from the lipid phase at below 100°C, preferably at 50 to 90°C.

11. Method according to one or more of claims 1 to 10, characterised in that the pH value of the aqueous phase is raised to between 2 and 8, preferably to between 3 and 7, by the addition of a basic compound which forms a salt of low solubility with an acid anion contained in the solution, this salt of low solubility preferably being Ca(OH)₂ and/or CaCO₃.

12. Method according to one or more of claims 1 to 11, characterised in that the solution remaining after separation of the precipitate is treated with a decolorant, preferably bentonite.

13. Method according to one or more of claims 1 to 12, characterised in that the solution which is chromatographed by way of the ion exchanger resin, is concentrated to provide a dry substance content of between 20 and 60% by weight, preferably between 25 and 50% by weight.

14. Method according to one or more of claims 1 to 13, characterised in that crystallisation of the rhamnose monohydrate is carried out as a crystallisation by cooling or by evaporation.

15. Method according to claim 14, characterised in that crystallisation by cooling is carried out at a cooling rate of between 1 and 10, preferably between 3 and 6°C per hour.

16. Method according to claim 14, characterised in that an oversaturation of between 1.05 and 1.3, preferably between 1.1 and 1.15, is maintained in the mother liquor during crystallisation by evaporation.

## Revendications

1. Procédé de fabrication de rhamnose à partir de rhamnolipides, **caractérisé** en ce qu'on hydrolyse une émulsion acide du rhamnolipide entre 100 et 200°C, après quoi on la refroidit, on sépare la phase aqueuse de l'hydrolysat formé de la phase lipide, on relève son pH par addition d'un composé basique, on sépare le précipité éventuel, on concentre la solution restante, ou bien on la transforme immédiatement, ou on la chromatographie sur une résine échangeuse d'ions, en obtenant sous la forme d'éluat les fractions contenant du rhamnose, ces fractions étant transformées telles quelles ou tranformées en monohydrate de rhamnose cristallin.

2. Procédé selon la revendication 1, caractérisé en ce qu'on utilise exclusivement l'eau comme solvant.

3. Procédé selon la revendication 1 ou 2, caractérisé en ce qu'on part d'une émulsion de rhamnolipides obtenue par fermentation.

4. Procédé selon une ou plusieurs des revendications 1 à 3, caractérisé en ce qu'on travaille en continu.

5. Procédé selon une ou plusieurs des revendications 1 à 4, caractérisé en ce que la phase lipide est recyclée dans le procédé, au plus tard dans l'opération d'hydrolyse.

6. Procédé selon une ou plusieurs des revendications 1 à 5, caractérisé en ce qu'on utilise une émulsion de rhamnolipide qui présente une conductivité inférieure à 12 mS/cm, de préférence inférieure à 5 mS/cm.

7. Procédé selon une ou plusieurs des revendications 1 à 6, caractérisé en ce que l'émulsion présente une teneur en matière sèche de 5 à 50% en poids, de préférence de 10 à 20% en poids.

8. Procédé selon une ou plusieurs des revendications 1 à 7, caractérisé en ce que la phase aqueuse de l'émulsion acide présente un pH de 0 à 3, de préférence de 0,5 à 1,5.

9. Procédé selon une ou plusieurs des revendications 1 à 8, caractérisé en ce que l'hydrolyse a lieu entre 110 et 160°C, de préférence entre 120 et 150°C.

10. Procédé selon une ou plusieurs des revendications 1 à 9, caractérisé en ce que la phase aqueuse de l'hydrolysat obtenu est séparée de la phase lipide à une température inférieure à 100°C, de préférence entre 50 et 90°C.

11. Procédé selon une ou plusieurs des revendications 1 à 10, caractérisé en ce qu'on relève le pH de la phase aqueuse par addition d'un composé basique qui forme un sel difficilement soluble avec un anion acide contenu dans la solution, de préférence Ca(OH)₂ et/ou CaCO₃, jusqu'à 2 à 8, de préférence entre 3 et 7.

12. Procédé selon une ou plusieurs des revendications 1 à 11, caractérisé en ce qu'on traite la solution restante après séparation du précipité avec un agent décolorant, de préférence de la bentonite.

13. Procédé selon une ou plusieurs des revendications 1 à 12, caractérisé en ce que la solution qui est chromatographiée sur la résine échangeuse d'ions est concentrée jusqu'à une teneur en matière sèche de 20 à 60% en poids, de préférence de 25 à 50% en poids.

14. Procédé selon une ou plusieurs des revendications 1 à 13, caractérisé en ce que la critallisation du monohydrate de rhamnose est réalisée par refroidissement ou par évaporation.

15. Procédé selon la revendication 14, caractérisé en ce que la cristallisation par refroidissement est réalisée à une vitesse de refroidissement de 1 à 10 °C/h, de préférence de 3 à 6 °C/h.

16. Procédé selon la revendication 14, caractérisé en ce qu'on maintient, lors de la cristallisation par évaporation, une sursaturation dans la liqueur-mère de 1,05 à 1,3, de préférence de 1,1 à 1,15.
